# EUROPEAN PATENT SPECIFICATION

(11) **EP 3 725 226 B1**
(45) Date of publication and mention of the grant of the patent: **20.07.2022**
(21) Application number: 18889383.8
(22) Date of filing: 10.09.2018
(51) Int. Cl.: A61B 5/15, A61B 5/151

(54) **PUSH BUTTON SAFETY ROTATION UNLOCKING-TYPE BLOOD COLLECTION DEVICE FOR INFANTS**
BLUTENTNAHMEVORRICHTUNG FÜR KLEINKINDER MIT DRUCKKNOPF-SICHERHEITSDREHLÖSUNG
DISPOSITIF DE COLLECTE DE SANG DE TYPE À DÉVERROUILLAGE PAR ROTATION DE SÉCURITÉ À BOUTON-POUSSOIR POUR NOURRISSONS

(30) Priority: 15.12.2017 CN 201711345727
(43) Date of publication of application: 21.10.2020
(73) Proprietor: Taicang Medlineer Medical Device Technology Co., Ltd., Taicang Suzhou, Jiangsu 215421 (CN)
(72) Inventor: LI, Gang, Suzhou, Jiangsu 215337 (CN)
(74) Representative: Kurig, Thomas
(86) International application number: PCT/CN2018/104839
(87) International publication number: WO 2019/114343

(56) References cited:
- CN-A- 105 105 766
- CN-A- 106 236 112
- CN-A- 107 951 493
- CN-U- 203 828 939
- US-A- 5 133 730

## Description

### BACKGROUND

### 1. TECHNICAL FIELD

The present invention relates to a medical blood collecting instruments, and more particularly to a cutting device which is usually used to controllably form a precise (or accurate) cutting (or wound/incision) in a patient's body surface. Specifically, it relates to a disposable cutting device which can be economically manufactured, have stable structure and reduce wrongly usage by the users. In this description, the device will be named as a lancing device for infant having a push-shooting button capable of safely rotating and unlocking. This lancing device for infant, which has a push-shooting button capable of safely rotating and unlocking, adopts a cutting type blood collecting method, which is mainly used to collect blood from infant's foot and can also be used to collect blood from other part of a body.

### 2. DESCRIPTION OF RELATED ART

Blood collecting devices or lancing devices are widely used to collect human blood in all kinds of medical units or hospitals, especially used for testing bleeding time or collecting blood sample. More important usage is used to form a cutting on skin of an infant foot and collect blood of the infant for evaluation of metabolic diseases or hereditary diseases. This kind of blood collecting method is suitable for newborns due to a quick movement of the blade.

A great variety of instruments for patient's skin cutting or puncture are sold in the market. The instruments, such as described in US Patent No. 4643189, US Patent No. 5133730 and Chinese Patent No. 100466973C, are employed to make a cutting on patient's skin with required length and depth.

US Patent No. 4643189 depicts a blood collecting device adopting compression spring to generate actuating energy. When the pre-loaded spring is released, the blade is driven to lance across the patient's skin and a precisely controlled cutting is then formed.

The spring of this kind of device is commonly pre-stretched or pre-compressed in order to accumulate energy to thereby actuate the blade (or make the blade movement) once the blood collecting device is triggered. However, it is harmful to stability of the blood collecting device due to such pre-stretched or pre-compressed. Especially, the pre-stretched or pre-compressed spring may result in undesired early or occasionally triggering or shooting. During assembly of the device, it also perhaps makes the worker suffering from injury because the elements may be occasionally pushed and ricocheted off by such spring. Besides, due to such pre-stretched or pre-compressed actions, other elements relating to the spring will maintain a high internal stress, which in turn results in shorter usage life of the device or poor performance.

It is an important factor to consider the manufacturing cost and/or the assembly cost, since the kind of device is designed for disposable usage. However, it is difficult to assemble the device when the spring is under pre-stretched or pre-compressed states. In some cases, special tools should be employed which result in that the manufacturing cost and the assembly cost are increased. Moreover, insurance structure should be added to prevent the blood collecting device from undesired shooting, such as secured button, blade cover. Obviously, the material cost, the manufacturing cost and the assembly cost are unavoidably increased.

The inventor thoroughly and repeatedly studies the conventional blood collecting devices and finds out the following problems. As for blood collecting devices, as introduced in US Patent No. 5133730, it adopts a method that the high polymer actuating arm is continuedly pressed, then deformed and finally released to thereby generate driven energy. No compressing spring is employed in such device. However, if the user occasionally presses the shooting triggering arm which is in a to-be-shoot state, that is, the shooting triggering arm is pushed inside of a shell, the shooting arm will pivot around and the blade is shooting out. Correspondingly, it will result in useless of the cutting device (this kind of device cannot be used).

In order to avoid undesired early shooting of the device, some products in the market, such as devices introduced in Chinese Patent No. 100466379C, a protecting cap connecting to a blade base is added to the front of the blade with a safety latch formed thereon. The safety latch is used to limit a shooting of the button. Only if the safety latch is removed away, the button can be normally triggered/shot. Although the protecting cap with safety latch resolve the undesired early shooting problem and protect the blade to be sterile and clean, shortcomings still exist. For example, the blade will be damaged when the blade protecting cap is pulled out inclinedly, which in turn increases pains during blood collecting. Further, the protecting cap is so small that easy to lose which will make the management of waste material inconvenience. It will be more dangerous if the protecting cap is picked up by kids who may put such protecting cap into his mouth.

Additionally, it exist defects when employing the continuedly pressing high polymer triggering arm method, in which the arm firstly deforms and then released. During producing stage, if the high polymer triggering arm is not pre-compressed and deformed in advance, the triggering arm has no enough striking force on the blade base when pressing and shooting, which will result in slowly cutting and make the pain increased. If the high polymer triggering arm is pre-compressed and deformed in advance, after a relatively long time of storage, the high polymer will perish which makes the pre-deformation lose the effectiveness and the triggering arm is ageing and curve.

According to the specific laws of some countries, each blood collecting device should be separatedly sealed up and encased, and the effective date, the standards, the manufacturing number should be all labeled on the outside of package. The steps of sealing up, packaging, sterilization make the blade be more sterile and clean, while the procedure is so complex that the user needs to remove the package first and then pull out the protecting cap, which in turn makes the value of the protecting cap decreased. Nevertheless, the safety latch attached on the protecting cap must be employed to maintain a safety use of the triggering/shooting button. It is desirable to design an improved device with/without blade protecting cap in order not to reduce the elements costs, but also to maintain stability performances.

Hence, based on the conventional technologies, the problems of how to design a low-cost device with long usage life and avoiding undesired shooting and how to design a package method with/without blade protecting cap, are all difficult issues for persons skilled in the art to resolve.

### SUMMARY

The present invention provides a lancing device for infant with a safely rotating and unlocking push-shooting button, which not only employs safety latch structure avoiding undesired shooting in advance, but also employs loaded shooting structure cooperating with the safety latch structure, so that one button structure can accomplish two performances and double effects are achieved step by step. The first objective of the present invention is to resolve problems of assembling difficulty and lifetime attenuation which is resulted from pre-loaded spring, so that, on one hand, the number of components of the device is decreased, on the other hand, the device can be loaded and applied at any time. The second objective of the present invention is to resolve the problem of safety latch.

In order to achieve above-mentioned objectives, the present invention provides a lancing device for infant with safety rotating and unlocking push-shooting button, which includes a shell and actuating lancing system that can be employed as one integral part or configured to consist of an edge lancing mechanism and a triggering and actuating mechanism. The edge lancing mechanism includes a blade and a blade base; or, the edge lancing mechanism is configured to be one integral element/part with a lancing edge. The triggering and actuating mechanism is a push-shooting triggering mechanism which includes a push-shooting button and an actuating arm. The push-shooting button is located on the shell and switches between two positions with respect to the shell by movement therebetween. The triggering and actuating mechanism is employed as one whole part rotatably connecting to the shell. The actuating arm is located inside the shell and the push-shooting button is exposed to outside of the shell opposite to the actuating arm. The one whole part includes a strip wafer connecting thereof and located at an intermediate area, which provides structural elastic for the one whole part. The push-shooting button occupies two kinds of position attitudes during a sliding path with respect to the shell, a first one attitude of which is an insurance lock-out state/position that the push-shooting button is locked to avoid an undesired trigger before actuation, and a second one attitude of which is a state that leaves away from the first one attitude where the push-shooting button is readily loaded to be launched/shot. During lancing, the push-shooting button is firstly locked in the insurance lock-out state/position and then rotated and loaded to a to-be-launch state/position; when the push-shooting button is pressed inwards, the blade base is actuated with the blade projecting from a lancing opening of the shell to make a V-shaped or an arc-shaped cut on a subject's skin and then retracted back into the shell. The whole blood collecting procedure is finished.

Statements and explanations on above-described disclosure are given below.
1. The "shell" refers to a covering structure consisting of two halves of shell interconnected together and provided for the lancing device for infant with safety rotating and unlocking push-shooting button. The "lancing mechanism" refers to a disposable cutting type lancing mechanism. The "insurance lock-out state" refers to an in-movable state when the push-shooting button is under pressing or striking in order to protect the lancing mechanism from occasionally shooting.
2. The actuating lancing system is a name for an assembly of the triggering and actuating mechanism, which is composed by the push-shooting button, the actuating arm and the elastic connecting strip wafer, and the edge lancing mechanism including the blade base and the blade. These functional modules can be assembled as one component/part either partially or totally. The pressing actuating element of the actuating lancing system may employ high polymer material with better elastic, such as nylon, POM, or optimized PC or ABS material. The blade can be made of metal and assembled to the high polymer blade base. Or, the blade and the blade base are integrally made from metal and are hollowed or adopts light metal material to thereby lose the weight. Even, the whole actuating lancing system can be integrally formed by metal.
3. Regarding issues of how to provisionally rotating and positioning of the actuating arm, the following method is provided: there is a provisional positioning structure is formed between the actuating arm and the shell during the rotating route changing from the insurance lock-out state to loaded to-be-launch state with respect to the shell. The provisional positioning structure is composed by a shaft arranged on one part and a shaft hole defined on other part. Since the position can be provisionally confirmed, the strip wafer in the middle of the triggering and actuating mechanism is bent and accumulates elastic energy, during rotating the push-shooting button from the insurance lock-out position to the actuating position.
4. There are two kinds of method for locating and detaching the triggering and actuating mechanism from the shell:
   the first kind of method: when pressing the push-shooting button under the shooting state, the releasable pushing block located in a distal end of the push-shooting button couples and squeezes with the actuating arm. There is a circle shaft hole is defined in the actuating arm aimed for provisional rotation. The shaft assembled on the shell has a small shaft height, and the distance from a top end of the shaft to the top of the shell is large. When the actuating arm is coupled and squeezed by the pushing block of the button, the actuating arm will slide and release from the shaft above the top end thereof;
   the second kind of method: there is a U-shaped hook provided on the actuating arm and the shaft on the shell has a large shaft height which connects the two opposite side walls of the shell. There is no possibility for the actuating arm detaching from the shaft. When the actuating arm is coupled and squeezed by the pushing block of the button, the actuating arm is bent along the pressing direction until the U-shaped hook released from lateral side of the shaft of the shell.
   the The advantages and positive effects of the present invention are introduced as follows:
      Firstly, the lancing device with the safely rotating and unlocking push-shooting button, not only employs safety locking structure avoiding undesired shooting in advance, but also employs loaded shooting structure cooperating with the safety latch structure, so that the button could work step by step which accomplish two performances and achieve double effects at the same time. The present invention does not need to use elements such as safety button or safety latch worked with the blade protecting cap which may result in undesired launching or shooting. The number of the elements for production is decreased and the costs of production and assembly are reduced. Additionally, the medical staff can use one hand operating at one controlling position to accomplish blood collecting procedure. Compared to conventional technology and conventional device, the present invention adopts one hand operation replacing two hands operation which improves the usage efficiency.
      Secondly, the push-shooting button provides, simultaneously, a safely rotating and locking operation together with a loaded to-be-shooting operation. The pre-pressed actuating force is generated. Compared to the conventional similar products, the present invention not only solves the problems on assembling difficulties and lifetime attenuations, but also reduces the usage on the spring components and the purchasing and assembling costs.

The foregoing has outlined rather broadly the features and technical advantages of the present invention in order that the detailed description of the invention that follows may be better understood. Additional features and advantages of the invention will be described hereinafter which form the subject of the claims of the invention.

### BRIEF DESCRIPTION OF THE DRAWINGS

The components in the drawing are not necessarily drawn to scale, the emphasis instead being placed upon clearly illustrating the principles of the described embodiments. In the drawings, reference numerals designate corresponding parts throughout various views, and all the views are schematic.
FIG. 1 is a view showing a push-shooting button of a lancing device according to the present invention under an insurance lock-out state/position.
FIG. 2 is a view showing the push-shooting button of the lancing device according to the present invention along a rotating and unlocking movement route.
FIG.3 is a view showing the push-shooting button of the lancing device according to the present invention under a loaded to-be-launch state after unlocking.
FIG. 4 is a view showing the lancing device of the present invention under a launched state.
FIG. 5 is a perspective view of FIG. 1.
FIG. 6 is a perspective view of FIG. 3 (showing a vertically guided locking structure).
FIG. 7 is a perspective view of FIG. 4 (showing a locking state after pressing and launching).
FIG. 8 is a partially enlarged view of FIG. 7 (showing a locking state after pressing and launching).
FIG. 9 is a perspective view of a lancing opening of the lancing device according to the present invention.
FIG. 10 is an internal view of the present lancing device showing a circle shaft hole of the actuating arm in accordance with one embodimentthe push-shooting button under an insurance lock-out state with partial shell being removed therefrom.
FIG. 11 is an internal view of the present lancing device showing a circle shaft hole of the actuating arm in accordance with one embodiment-the push-shooting button under the loaded to-be-launch state after unlocking with partial shell being removed therefrom.
FIG. 12 is an internal view of the present lancing device showing a circle shaft hole of the actuating arm in accordance with one embodimentthe push-shooting button under the launched state with partial shell being removed therefrom.
FIG. 13 is a partial perspective view of the present lancing device showing a circle shaft hole of the actuating arm in accordance with one embodiment ---- the locked actuating arm under the loaded to-be-launch state.
FIG. 14 is a partial perspective view of the present lancing device showing a circle shaft hole of the actuating arm in accordance with one embodiment ---- the unlocked actuating arm under a shooting state.
FIG. 15 is an internal view of the present lancing device showing a U-shaped hook of the actuating arm in accordance with the other embodiment ---- the push-shooting button under an insurance lock-out state with partial shell being removed therefrom.
FIG. 16 is an internal view of the present lancing device showing the U-shaped hook of the actuating arm in accordance with the other embodiment ---- the push-shooting button under the loaded to-be-launch state after unlocking with partial shell being removed therefrom.
FIG. 17 is a partial perspective view of the present lancing device showing the U-shaped hook of the actuating arm in accordance with the other embodiment ---- the locked actuating arm under the loaded to-be-launch state.
FIG. 18 is a view showing a blood collecting moving route of the blade of the present lancing device with partial shell being removed therefrom.
FIG. 19 is a view showing the present lancing device with a blade protecting hood.
FIG. 20 is an exploded perspective view of the present lancing device.
FIG. 21 is a view showing a triggering and actuating mechanism of the present lancing device.
FIG. 22 is a view showing an edge lancing mechanism of the present lancing device.
FIG. 23 is a view showing the one-piece triggering and actuating lancing system used in the present lancing device.

In these drawings: 1. Shell; 2. Push-shooting button; 3. Actuating arm; 5. Blade base; 6. Rotation shaft; 7. Circle shaft hole; 8. U-shaped hook; 9. Strip wafer; 10. Unlocking guiding slot; 11. Blade edge lancing guiding slot; 12. Actuating guiding post; 13. releasable pushing block; 14. lancing guiding protrusion; 15. actuated striking block; 16. Balance controlling protrusion; 17. anti-ejecting lump; 18. anti-ejecting barb; 19. Shooting-guided slot; 20. Shooting and pressing surface; 21. Lancing opening; 22. Blade-protecting hood.

### DETAILED DESCRIPTION OF THE ILLUSTRATED EMBODIMENT

Reference will now be made to the drawing figures to describe the embodiments of the present disclosure in detail. In the following description, the same drawing reference numerals are used for the same elements in different drawings.

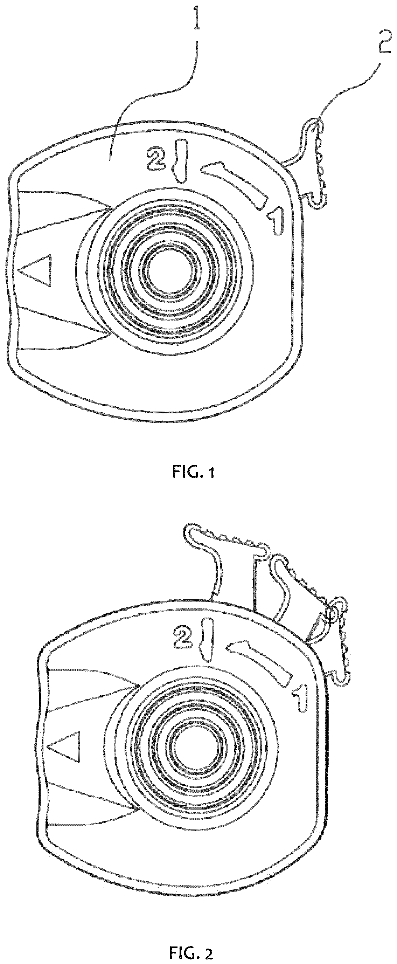

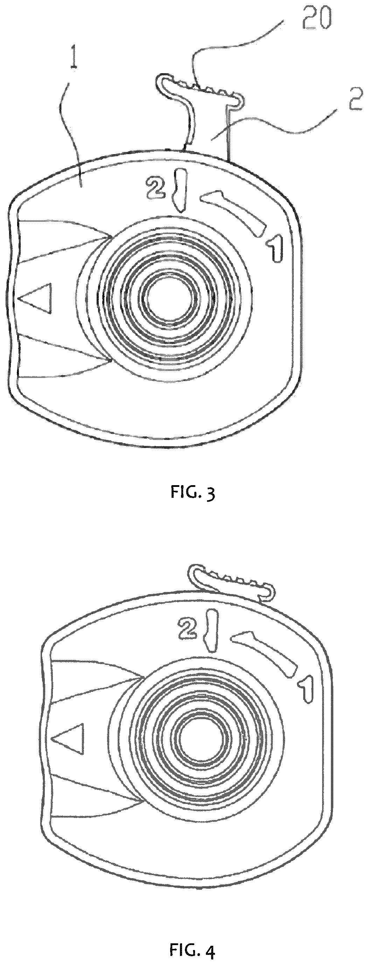

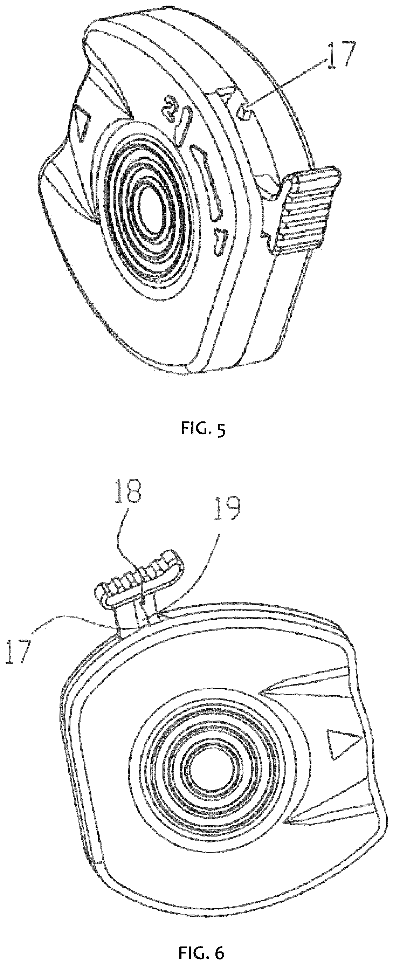

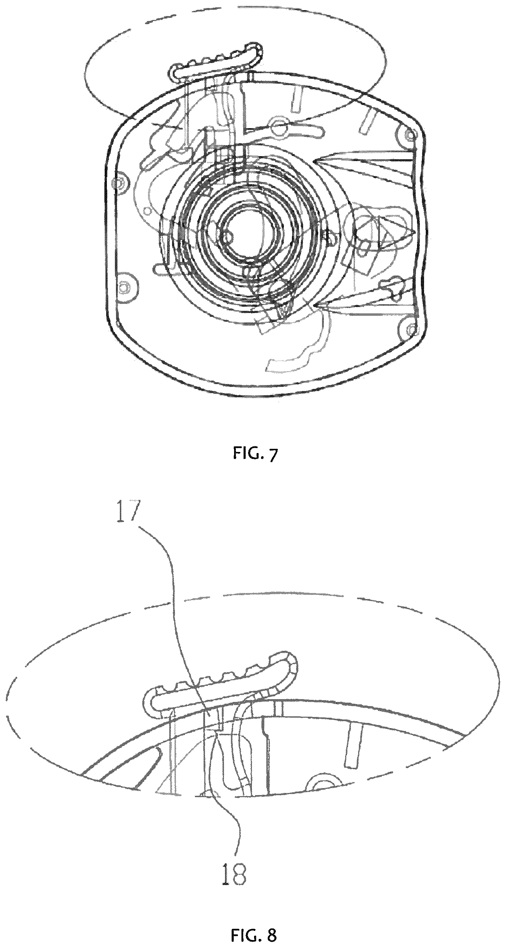

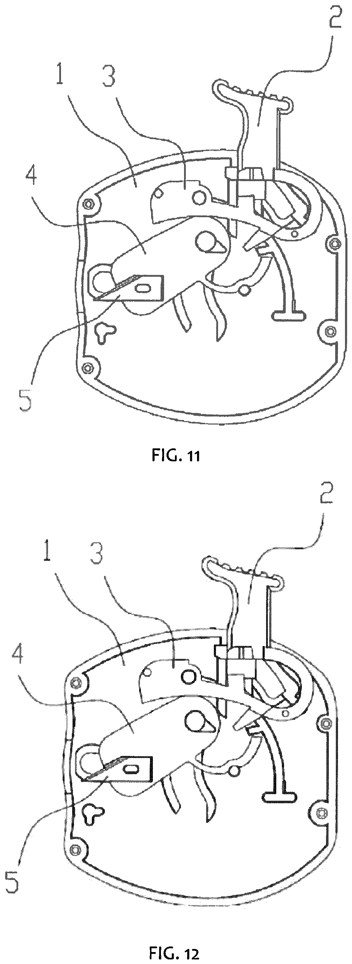

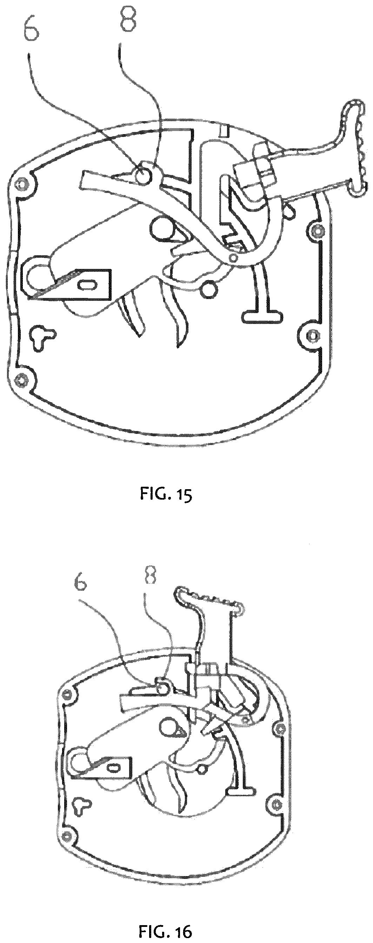

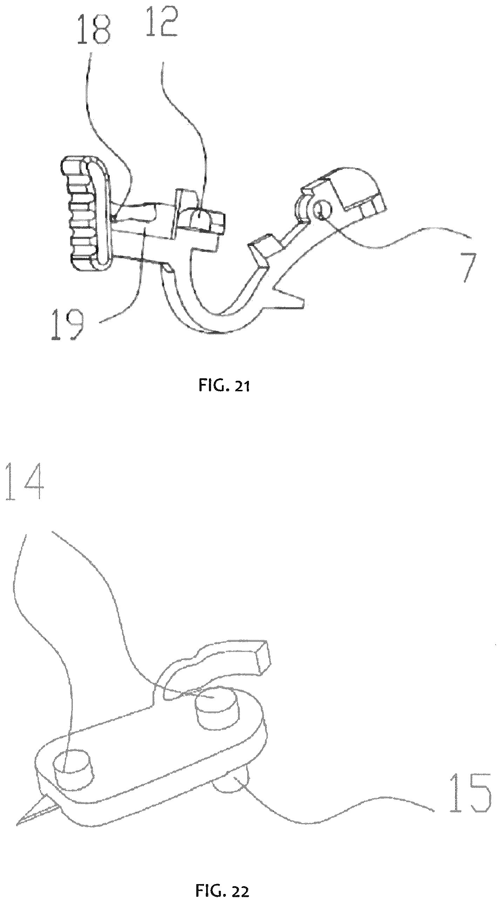

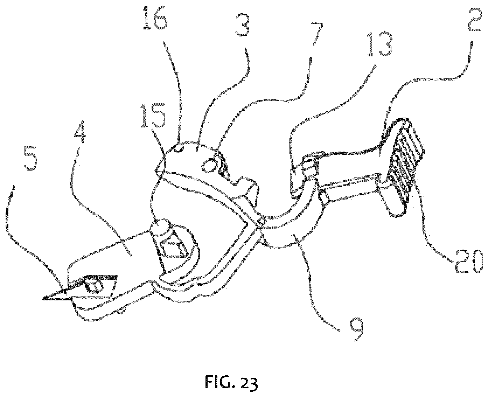

Referring to FIGS. 1 to 23, an illustrated embodiment of the present disclosure discloses a lancing device for infant with safety rotating and unlocking push-shooting button. The lancing device includes a shell 1 and an actuating lancing system that can be employed as one integral part or configured to consist of a triggering and actuating mechanism (FIG. 21) and an edge lancing mechanism (FIG. 22). The edge lancing mechanism includes a blade 4 and a blade base 5. Or, referring to FIG. 23, the edge lancing mechanism is configured to be one integral element/part with a lancing edge. The triggering and actuating mechanism is a push-shooting structure which includes a push-shooting button 2 and an actuating arm 3. The push-shooting button 2 is located on the shell 1 and switches between two positions with respect to the shell 1 by movement therebetween. The triggering and actuating mechanism is employed as one whole part rotatably connecting to the shell. The actuating arm 3 is located inside of the shell 1 and the push-shooting button 2 is exposed to outside of the shell 1 opposite to the actuating arm 3. The push-shooting button 2 occupies two kinds of position attitudes/states during a sliding path/route with respect to the shell 1, a first one attitude of which is an insurance lock-out state/position that the push-shooting button 2 is locked to avoid an undesired trigger before actuation (as shown in FIG. 1 and FIG. 5), and a second one attitude of which is a state that leaves away from the first one attitude where the push-shooting button 2 is readily loaded to be launched/shot (as shown in FIG. 3 and FIG. 6). Under the insurance lock-out state, the push-shooting button 2 is locked at a distal end so that the pressing operation/action cannot make the push-shooting button 2 move/displace. Only if a rotation operation/action is exerted, the push-shooting button 2 can move. During lancing, the push-shooting button 2 is firstly locked in the insurance lock-out state/position and then rotated and loaded to a to-be-launch state/position (as shown in FIG. 2); when the push-shooting button 2 is pressed inwards, the blade base 5 is actuated with the blade 4 projecting from a lancing opening 21 of the shell 1 to make a V-shaped or an arc-shaped cut on a subject's skin and then retracted back into the shell 1. The whole blood collecting procedure is finished.

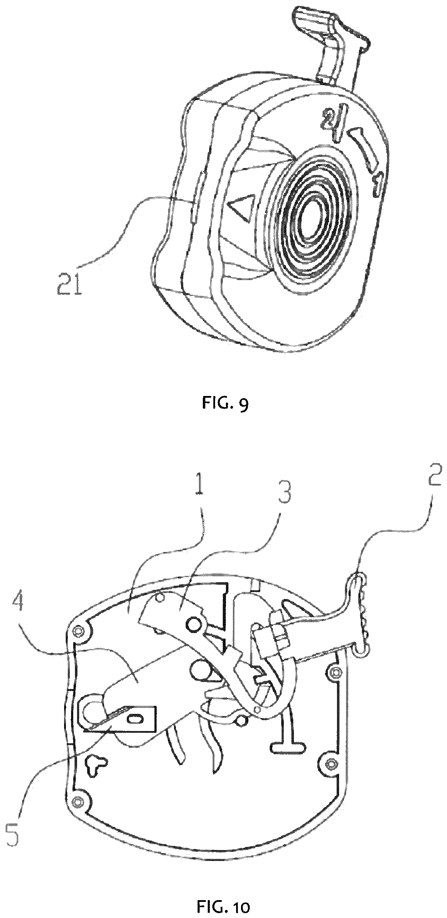

Referring to FIGS. 10, 13, 15 and 21, the triggering and actuating mechanism is employed as one whole part/element rotatably connecting to the shell 1. The actuating arm 3 defines a provisional connecting structure thereon and rotatably connects to inside wall of the shell 1 around a rotation shaft 6 through an engagement between the rotation shaft 6 and a shaft hole 7. In the other embodiment, the rotation shaft 6 is engageable with a U-shaped hook 8. The push-shooting button 2 has one rigid end exposed outsides from the shell 1. The one whole element includes a strip wafer 9 connecting the push-shooting button 2 with the actuating arm 3. The strip wafer 9 is arranged at an intermediate area of the triggering and actuating mechanism in order to provide structural elastic for the whole element. When the push-shooting button 2 is rotated from the insurance lock-out position to the triggering and launching position, the intermediate strip wafer 9 is deformed and generates elastic potential energy therein due to a distance between the push-shooting button and the actuating arm changing from large to small. The elastic potential energy is released to actuate the edge lancing mechanism movement when the actuating arm 3 is suddenly released at the launching position.

Referring to FIGS. 2 and 21, the triggering and actuating mechanism employs an actuating guiding post 12 arranged beside the push-shooting button 2. The actuating guiding post 12 engages with an unlocking guiding slot 10 of the shell 1 to change the states/positions of the push-shooting button 2 along the sliding path thereof. A length that the push-shooting button 2 exposed to outside or a distance between the push-shooting button 2 and the shell 3 is changeable because the unlocking guiding slot 10 is such configured that a distance from the unlocking guiding slot 10 to a periphery of the shell 1 is unequal. The length that the push-shooting button 2 exposed to outside is short and is difficult to be touched under the insurance lock-out state, while the length is long under the loaded to-be-launched state which provides enough pressing journey for the button 2 to thereby make the operation/action more comfortable.

Referring to FIG. 6, according to the disposable lancing device of the present invention, when the push-shooting button 2 of the lancing device of the present invention moves from the first position attitude/state to the second position attitude/state along the sliding path/route with respect to the shell 1, the strip wafer 9 is bent and exerts backwards force onto the button 2 to return to the first position attitude/state. The engagement between the anti-ejecting lump 17 and an anti-ejecting slot or a shooting guiding slot 19, secures the push-shooting button 2 on a loaded to-be-launched state along a vertical direction.

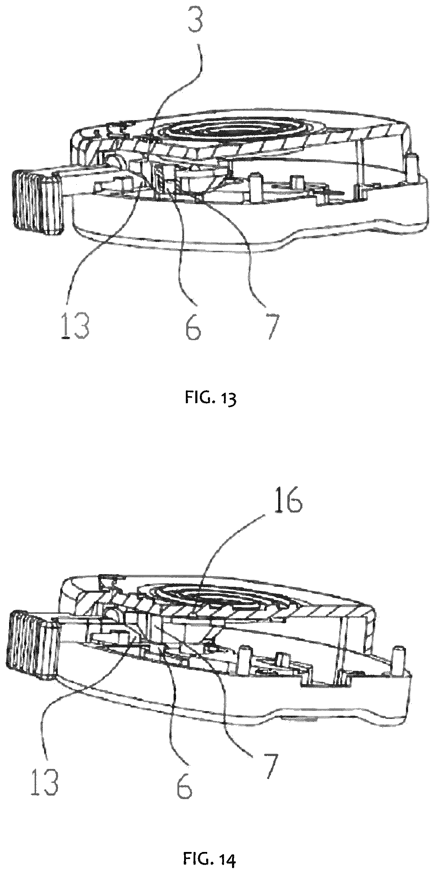

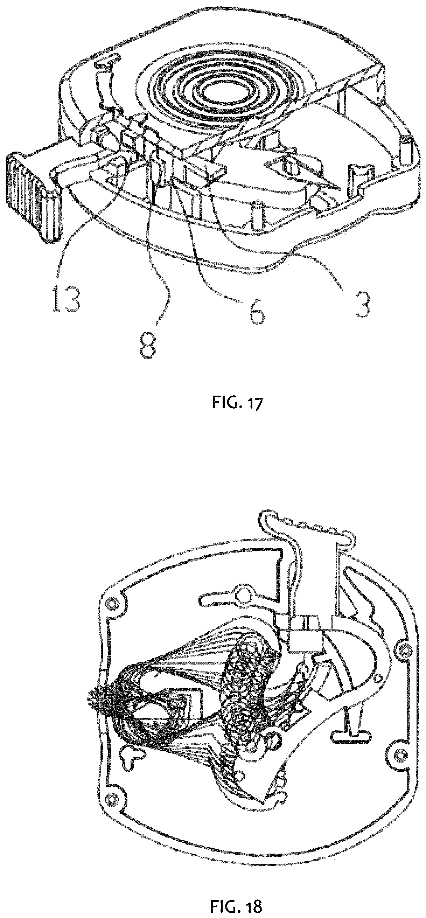

Referring to FIGS. 13 and 14, according to the disposable lancing device of the present invention, the push-shooting button 2 of the triggering and actuating mechanism employs a releasable pushing block 13 at a distal end thereof. When the push-shooting button 2 is pressed under the to-be-launch state, the releasable pushing block 13 squeezes and couples with the actuating arm 3 so as to release the circle shaft hole 7 or the U-shaped hook from the rotation shaft 6 and actuating energy is thereby generated. In the one embodiment, the circle shaft hole 7 is provided and employed on the actuating arm 3. The shaft 6 assembled on the shell 1 has a small shaft height, and the distance from a top end of the shaft 6 to the top of the shell 1 is large. When the actuating arm is coupled and squeezed by the pushing block 13 of the button 2, the distal end of the actuating arm 3 is bent and deformed with a balance controlling protrusion 16 formed on the arm 3 subjecting to a vertical force. The force along the pressing direction and the force along a direction vertical to the pressing direction work together in order to drive the shaft hole 7 sliding and finally releasing from the shaft 6 across the top end thereof. In the other embodiment, referring to FIGS. 17, the U-shaped hook 8 is provided and employed on the actuating arm 3. The shaft 6 on the shell 1 has a large shaft height which connects two opposite side walls of the shell 1. When the actuating arm 3 is coupled and squeezed by the pushing block 13 of the button 2, the actuating arm 3 is bent along the pressing direction until the U-shaped hook 8 released from lateral side of the shaft 6 of the shell 1.

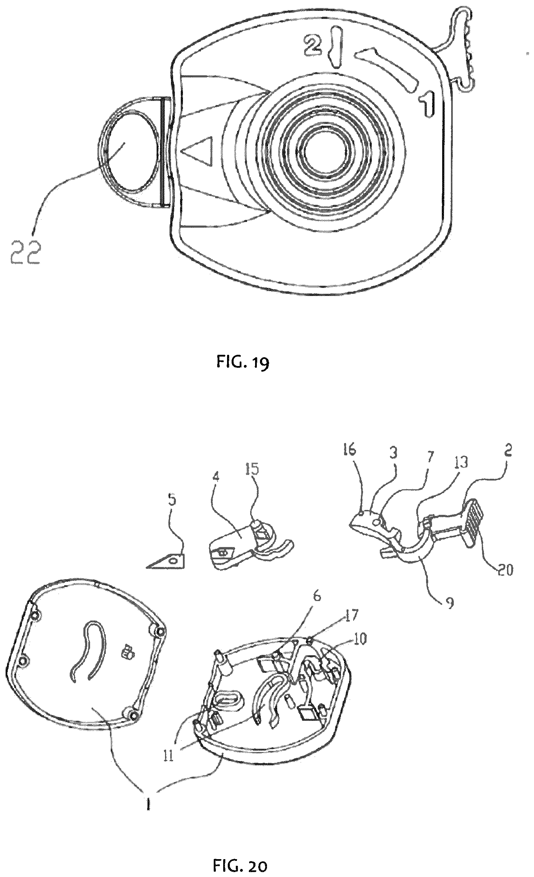

Referring to FIG. 20, the blade edge lancing mechanism comprises a pair of lancing guiding protrusions 14 and an actuated striking block 15. The pair of lancing guiding protrusions 14 are engageable with the pair of blade edge lancing guiding slots 11, respectively. One of the blade edge lancing guiding slots 11 is defined to have a shape approximate to a curve and the other one of the blade edge lancing guiding slots is defined to have a shape approximate to a straight line. When the actuating arm 3 of the triggering and actuating mechanism is released, the elastic potential energy makes the actuating arm 3 push the actuated striking block 15 of the blade edge lancing mechanism to move. One of the lancing guiding protrusions 14 moves along a curved path in the curved guiding slot 11, and the other one of the lancing guiding protrusions 14 moves forwards and then backwards in the straight line shape guiding slot 11. As can be understood, the rotating movement of the actuating arm 3 turns into a rotation and a translation of the blade 4. Consequently, the blade 4 makes an arced or V-shaped cutting and then retracts back into the shell 1. The blood collecting procedure is finished.

Referring to FIGS. 5-8, the push-shooting button 2 has an anti-ejecting barb 18 formed thereon. The shell 1 has an anti-ejecting lump 17 formed thereon for engaging and locking with the anti-ejecting barb 18. When the launching action of the push-shooting button 2 is accomplished, the barb 18 and the lump 17 interlocks with each other to thereby prevent the push-shooting button 2 back to an initial to-be-shoot state/position. At this situation, the blade 4 is restricted and stopped by the front end of the actuating arm 3. The blade is unable to move so as to avoid the blade bringing therewith blood from cutting the body which will result in second time damage and crossover infection. Moreover, it is convenient for the user to recognize the status of the button 2 after pressing and locking the button 2.

Referring to FIG. 19, a blade-protecting hood 22 is selectively provided, which can be tore open or snapped off to thereby detach from the blade 4. The blade-protecting hood 22 helps to maintain the sharpness of the blade 4 and make the blade 4 sterile and clean. The employment of such blade protecting hood 22 enable the lancing device to satisfy rules or habits of some countries.

It is to be understood, however, that even though numerous characteristics and advantages of preferred and exemplary embodiments have been set out in the foregoing description, together with details of the structures and functions of the embodiments, the disclosure is illustrative only; and that changes may be made in detail within the principles of present disclosure to the full extent indicated by the broadest general meaning of the terms in which the appended claims are expressed.

## Claims

1. A lancing device for infant with an actuating button adapted for safely rotating and unlocking, comprising a shell (1) and an actuating lancing system;
said actuating lancing system comprising a triggering and actuating mechanism and an edge lancing mechanism;
said edge lancing mechanism having a blade (4) and a blade base (5); or, said edge lancing mechanism formed as one integral element with lancing edge;
said triggering and actuating mechanism is a pushing actuating-type structure, including a push-shooting button (2) and an actuating arm (3), said push-shooting button (2) slidably located on said shell (1) and switching between two positions with respect to said shell (1) by movement therebetween;
wherein said push-shooting button (2) is exposed to outside of said shell (1), said push-shooting button (2) occupies two kinds of position attitudes during a sliding path with respect to said shell (1); a first one attitude is an insurance lock-out state/position that said push-shooting button (2) is locked to avoid an undesired trigger before actuation, and a second one attitude is a state that leaves away from the first one attitude where said push-shooting button (2) is readily loaded to-be-launched/shot; during lancing, said push-shooting button (2) is firstly locked in the insurance lock-out state/position and then rotated and loaded to a to-be-launch state/position; when the push-shooting button (2) is pressed inwards, said blade base (5) is actuated with said blade (4) projecting from a lancing opening (21) of said shell to make a V-shaped or an arc-shaped cut on a subject's skin and then retracted back into said shell.

2. The lancing device as claimed in claim 1, wherein said triggering and actuating mechanism works as one integral component which is rotatably connecting to said shell (1), said one integral component has an actuating arm (3) formed therein, said actuating arm (3) defines a provisional connecting structure thereon and rotatably connects to inside wall of said shell (1) around a rotation shaft (6) through an engagement between said rotation shaft and a shaft hole (7) or an engagement between said rotation shaft (6) and a U-shaped hook (8); wherein said push-shooting button (2) has one rigid end exposed outsides from said shell, and said one integral component includes a strip wafer (9) connecting thereof and located at an intermediate area thereof in order to provide structural elastic for said one integral component; wherein when said push-shooting button (2) is rotated from said insurance lock-out position to said triggering and launching position, said intermediate strip wafer (9), connecting said push-shooting button (2) with said actuating arm (3), is deformed and generates elastic potential energy therein due to a distance between said push-shooting button (2) and said actuating arm (3) changing from large to small; and wherein said elastic potential energy is released to actuate said edge lancing mechanism movement when said actuating arm (3) is suddenly released at said launching position.

3. The lancing device as claimed in claim 1, wherein said triggering and actuating mechanism and said edge lancing mechanism of said actuating lancing system are either worked as one whole part or worked as two separated elements.

4. The lancing device as claimed in claim 1, wherein said shell has an inside wall defining an unlocking guiding slot used for said actuating button and a pair of blade edge lancing guiding slot; wherein said unlocking guiding slot is adapted to guide said actuating button slidable moving therein and switching between said insurance lock-out state/position and said to-be-launch state/position; and wherein said blade edge lancing guiding slot leads said blade edge lancing mechanism moving therealong so as to make said blade extend outward from said lancing opening of said shell and generate a V-shaped or an arc-shaped cut.

5. The lancing device as claimed in claim 4, wherein said triggering and actuating mechanism employs an actuating guiding post arranged beside said push-shooting button, which engages with said unlocking guiding slot of said shell to change said states/positions of said push-shooting button along said sliding path; and wherein a length that said push-shooting button exposed to outside or a distance between said push-shooting button and said shell is changeable because said unlocking guiding slot is such configured that a distance from said unlocking guiding slot to a periphery of said shell is unequal; and wherein said length that said push-shooting button exposed to outside is short and difficult to be touched during said insurance lock-out state, while said length is long during said to-be-launched state.

6. The lancing device as claimed in any one of claims 1-4, wherein said push-shooting button of said triggering and actuating mechanism employs a releasable pushing block at a distal end thereof; wherein when said push-shooting button is pressed in said to-be-launch state, said releasable pushing block squeezes and couples with said actuating arm so as to release said shaft hole or said U-shaped hook from said rotation shaft and actuating energy is thereby generated.

7. The lancing device as claimed in any one of claims 1-6, wherein said blade edge lancing mechanism comprises a pair of lancing guiding protrusions and an actuated striking block, said pair of lancing guiding protrusions engaging with said pair of blade edge lancing guiding slots, respectively; wherein one of said blade edge lancing guiding slots is defined to have a shape approximate to a curve and the other one of said blade edge lancing guiding slots is defined to have a shape approximate to a straight line; and wherein when said actuating arm of said triggering and actuating mechanism is released, said elastic potential energy makes said actuating arm push said actuated striking block of said blade edge lancing mechanism to move, and subsequently, said pair of lancing guiding protrusions are driven to engage and move in corresponding blade edge lancing guiding slots, so that a rotation and a translation of said blade are fulfilled and finished.

8. The lancing device as claimed in any one of claims 1-7, wherein said push-shooting button has an anti-ejecting barb formed thereon and said shell has an anti-ejecting lump formed thereon for engaging and locking with said anti-ejecting barb, when said launching action of said push-shooting button is accomplished.

9. The lancing device as claimed in any one of claims 1-8, further comprising a blade-protecting hood, which can be tore open or snapped off to thereby detach from said blade.

## Patentansprüche

1. Stechvorrichtung für Kleinkind mit einem Betätigungsknopf, der für sicheres Drehen und Entriegeln angepasst ist, umfassend ein Gehäuse (1) und ein Betätigungsstechsystem;
wobei das Betätigungsstechsystem einen Auslöse- und Betätigungsmechanismus und einen Kantenstechmechanismus umfasst;
wobei der Kantenstechmechanismus eine Schneide (4) und eine Schneidbasis (5) aufweist; oder der Kantenstechmechanismus als ein integrales Element mit Stechkante gebildet ist;
der Auslöse- und Betätigungsmechanismus eine drückbetätigbare Struktur ist, die einen Druckschussknopf (2) und einen Betätigungsarm (3) einschließt, wobei sich der Druckschussknopf (2) verschiebbar an dem Gehäuse (1) befindet und zwischen zwei Positionen durch Bewegung dazwischen in Bezug auf das Gehäuse (1) wechselt;
wobei der Druckschussknopf (2) zur Außenseite des Gehäuses (1) freiliegt, wobei der Druckschussknopf (2) während eines Gleitweges in Bezug auf das Gehäuse (1) zwei Arten von Positionseinstellungen einnimmt; wobei eine erste Einstellung einen Sicherungs-Verriegelungszustand / eine Sicherungs-Verriegelungsposition ist, in dem / in der der Druckschussknopf (2) verriegelt ist, um ein unerwünschtes Auslösen vor Betätigung zu vermeiden, und eine zweite Einstellung ein Zustand ist, der von der ersten Einstellung abweicht, in der der Druckschussknopf (2) betriebsbereit geladen ist, um gestartet / ausgelöst zu werden; wobei, während Stechens, der Druckschussknopf (2) zunächst in dem Sicherungs-Verriegelungszustand / in der Sicherungs-Verriegelungsposition verriegelt und dann gedreht und in einem zu startenden Zustand / eine Auslöseposition geladen wird; wobei, wenn der Druckschussknopf (2) nach innen gedrückt wird, die Schneidbasis (5) mit der Schneide (4), die aus einer Stechöffnung (21) des Gehäuses vorsteht, betätigt wird, um einen V-förmigen oder bogenförmigen Schnitt auf der Haut einer Person zu machen, und dann in das Gehäuse zurückgezogen wird.

2. Stechvorrichtung wie beansprucht in Anspruch 1, wobei der Auslöse- und Betätigungsmechanismus als ein integrales Bauteil arbeitet, das drehbar mit dem Gehäuse (1) verbunden ist, wobei das eine integrale Bauteil einen darin gebildeten Betätigungsarm (3) aufweist, wobei der Betätigungsarm (3) eine provisorische Verbindungsstruktur darauf definiert und drehbar mit der Innenwand des Gehäuses (1) um eine Drehwelle (6) durch einen Eingriff zwischen der Drehwelle und einem Wellenloch (7) oder einen Eingriff zwischen der Drehwelle (6) und einem U-förmigen Haken (8) verbunden ist;
wobei der Druckschussknopf (2) ein starres Ende aufweist, das außerhalb des Gehäuses freiliegt, und das eine integrale Bauteil einen Streifenwafer (9) einschließt, der hiervon verbindet und sich in einem Zwischenbereich hiervon befindet, um strukturelle Elastizität für das eine integrale Bauteil bereitzustellen; wobei, wenn der Druckschussknopf (2) von der Sicherungs-Verriegelungsposition in die Auslöse- und Startposition gedreht wird, der Zwischenstreifenwafer (9), der den Druckschussknopf (2) mit dem Betätigungsarm (3) verbindet, verformt wird und darin elastische potentielle Energie aufgrund eines Abstands zwischen dem Druckschussknopf (2) und dem Betätigungsarm (3) erzeugt, der sich von groß zu klein ändert; und wobei die elastische potentielle Energie freigesetzt wird, um Kantenstechmechanismus-Bewegung zu betätigen, wenn der Betätigungsarm (3) in der Startposition plötzlich freigegeben wird.

3. Stechvorrichtung wie beansprucht in Anspruch 1, wobei der Auslöse- und Betätigungsmechanismus und der Kantenstechmechanismus des betätigenden Stechsystems entweder als ein ganzes Teil gearbeitet sind oder als zwei getrennte Elemente gearbeitet sind.

4. Stechvorrichtung wie beansprucht in Anspruch 1, wobei das Gehäuse eine Innenwand, die einen Entriegelungs-Führungsschlitz aufweist, der für den Betätigungsknopf verwendet wird, und ein Paar Schneidkanten-Stechführungsschlitze definiert; wobei der Entriegelungs-Führungsschlitz angepasst ist, den Betätigungsknopf zu führen, der sich darin gleitend bewegt und zwischen dem Sicherungs-Verriegelungszustand / der Sicherungs-Verriegelungsposition und dem zu startenden Zustand / der Auslöseposition wechselt; und wobei der Schneidkanten-Stechführungsschlitz dazu führt, dass sich der Schneidkanten-Stechmechanismus daran entlang bewegt, so dass sich die Schneide von der Stechöffnung des Gehäuses nach außen erstreckt und einen V-förmigen oder einen bogenförmigen Schnitt erzeugt.

5. Stechvorrichtung wie beansprucht in Anspruch 4, wobei der Auslöse- und Betätigungsmechanismus einen neben dem Druckschussknopf angeordneten Betätigungsführungsstift verwendet, der in den Entriegelungs-Führungsschlitz des Gehäuses eingreift, um die Zustände / Positionen des Druckschussknopfes entlang des Gleitweges zu ändern; und wobei eine Länge, die der Druckschussknopf zur Außenseite hin freiliegt oder ein Abstand zwischen dem Druckschussknopf und dem Gehäuse veränderbar ist, weil der Entriegelungs-Führungsschlitz so konfiguriert ist, dass ein Abstand von dem Entriegelungs-Führungsschlitz zu einem Randbereich des Gehäuses ungleich ist; und wobei die Länge, die der Druckschussknopf zur Außenseite hin freiliegt, während des Sicherungs-Verriegelungszustands kurz und schwierig zu berühren ist, während die Länge während des zu startenden Zustands lang ist.

6. Stechvorrichtung wie beansprucht in einem der Ansprüche 1-4, wobei der Druckschussknopf des Auslöse- und Betätigungsmechanismus einen lösbaren Druckblock an einem distalen Ende davon verwendet; wobei, wenn der Druckschussknopf in dem zu startenden Zustand gedrückt wird, der lösbare Druckblock den Betätigungsarm zusammendrückt und mit ihm koppelt, um das Wellenloch oder den U-förmigen Haken von der Drehwelle zu lösen, und Betätigungsenergie dadurch erzeugt wird.

7. Stechvorrichtung wie beansprucht in einem der Ansprüche 1-6, wobei der Schneidkanten-Stechmechanismus ein Paar von Stechführungsvorsprüngen und einen betätigten Anschlagblock umfasst, wobei das Paar von Stechführungsvorsprüngen jeweils in das Paar von Schneidkanten-Stechführungsschlitze eingreift; wobei einer der Schneidkanten-Stechführungsschlitze so definiert ist, dass er eine Form aufweist, die ungefähr einer Kurve entspricht, und der andere der Schneidkanten-Stechführungsschlitze so definiert ist, dass er eine Form aufweist, die ungefähr einer geraden Linie entspricht; und wobei, wenn der Betätigungsarm des Auslöse- und Betätigungsmechanismus gelöst wird, die elastische potentielle Energie den Betätigungsarm veranlasst, den betätigten Anschlagblock des Schneidkanten-Stechmechanismus zu bewegen, und anschließend das Paar von Stechführungsvorsprüngen angetrieben wird, um in entsprechende Schneidkanten-Stechführungsschlitze einzugreifen und sich darin zu bewegen, so dass eine Drehung und eine Translation der Schneide erfüllt und beendet werden.

8. Stechvorrichtung wie beansprucht in einem der Ansprüche 1-7, wobei der Druckschussknopf einen darauf gebildeten Antiauswurfwiderhaken aufweist und das Gehäuse ein darauf gebildetes Antiauswurfstück aufweist, um mit dem Antiauswurfwiderhaken einzugreifen und diesen zu verriegeln, wenn die Starthandlung des Druckschussknopfes vollendet ist.

9. Stechvorrichtung wie beansprucht in einem der Ansprüche 1-8, ferner umfassend eine Schneidschutzhaube, die aufgerissen oder abgeknickt werden kann, um sich dadurch von der Schneide zu lösen.

## Revendications

1. Un dispositif de piqûre pour nourrisson avec un bouton d'actionnement adapté pour une rotation et un déverrouillage en toute sécurité, comprenant une coque (1) et un système d'actionnement de piqûre ;
ledit système d'actionnement de piqûre comprenant un mécanisme de déclenchement et d'actionnement et un mécanisme de piqûre situé en bordure ;
ledit mécanisme de piqûre situé en bordure ayant une lame (4) et une base de lame (5) ; ou, ledit mécanisme de piqûre situé en bordure étant sous la forme d'un élément en une pièce avec un bord de piqûre ;
ledit mécanisme de déclenchement et d'actionnement est une structure de type à actionnement par poussée, comprenant un bouton-poussoir (2) de déclenchement et un bras d'actionnement (3), ledit bouton-poussoir (2) de déclenchement étant situé de manière coulissante sur ladite coque (1) et étant déplaçable entre deux positions par rapport à ladite coque (1), par déplacement entre celles-ci ;
ledit bouton-poussoir (2) de déclenchement étant exposé à l'extérieur de ladite coque (1), ledit bouton-poussoir (2) de déclenchement occupant deux types de positionnements lors d'un trajet de coulissement par rapport à ladite coque (1) ; un premier positionnement est un état/une position de verrouillage assuré(e) dans lequel/laquelle ledit bouton-poussoir (2) de déclenchement est verrouillé de façon à éviter un déclenchement indésirable avant l'actionnement, et un deuxième positionnement est un état qui s'éloigne du premier positionnement, dans lequel ledit le bouton-poussoir (2) de déclenchement est facilement chargé pour être déclenché/activé ; pendant la piqûre, ledit bouton-poussoir (2) de déclenchement est d'abord verrouillé dans l'état/la position de verrouillage assuré(e), puis est tourné et chargé dans un état/une position de déclenchement ; lorsque le bouton-poussoir (2) de déclenchement est enfoncé vers l'intérieur, ladite base de lame (5) est actionnée avec ladite lame (4) de façon à faire saillie à partir d'une ouverture de piqûre (21) de ladite coque afin d'effectuer une coupe en forme de V ou en forme d'arc sur la peau d'un sujet puis à être rétractée dans ladite coque.

2. Le dispositif de piqûre selon la revendication 1, dans lequel ledit mécanisme de déclenchement et d'actionnement fonctionne comme un composant en une pièce qui est relié de manière rotative à ladite coque (1), ledit un composant en une pièce ayant un bras d'actionnement (3) formé à l'intérieur de lui, ledit bras d'actionnement (3) définissant une structure de connexion provisoire sur lui et se connectant de manière rotative à la paroi intérieure de ladite coque (1) autour d'un arbre de rotation (6) par un engagement entre ledit arbre de rotation et un trou d'arbre (7) ou par un engagement entre ledit arbre de rotation (6) et un crochet (8) en forme de U;
ledit bouton-poussoir (2) de déclenchement a une extrémité rigide exposée à l'extérieur de ladite coque, et ledit un composant en une pièce comprend une plaquette (9) sous forme de bande le reliant et située dans une zone intermédiaire de celui-ci afin de fournir un élastique structurel pour ledit un composant en une pièce ; lorsque ledit bouton-poussoir (2) de déclenchement est tourné de ladite position de verrouillage assurée à ladite position de déclenchement et de piqûre, ladite plaquette intermédiaire (9) sous forme de bande, reliant ledit bouton-poussoir (2) de déclenchement audit bras d'actionnement (3), est déformée et génère une énergie potentielle élastique en elle en raison d'une distance entre ledit bouton-poussoir (2) de déclenchement et ledit bras d'actionnement (3), qui passe de grande à petite ; et ladite énergie potentielle élastique est libérée de façon à actionner ledit mouvement du mécanisme de piqûre situé en bordure lorsque ledit bras d'actionnement (3) est soudainement relâché à ladite position de déclenchement.

3. Le dispositif de piqûre selon la revendication 1, dans lequel ledit mécanisme de déclenchement et d'actionnement et ledit mécanisme de piqûre situé en bordure dudit système de piqûre d'actionnement sont soit façonnés sous la forme une pièce entière, soit façonnés sous la forme deux éléments séparés.

4. Le dispositif de piqûre selon la revendications 1, **caractérisé en ce que** ladite coque présente une paroi interne définissant une fente de guidage de déverrouillage servant audit bouton d'actionnement et une paire de fentes de guidage de piqûre en bordure pour lame ; ladite fente de guidage de déverrouillage est adaptée pour guider ledit bouton d'actionnement coulissant qui est déplaçable en elle et qui se déplace entre ledit état/ladite position de verrouillage assuré(e) et ledit état/ladite position de déclenchement ; et ladite fente de guidage de piqûre en bordure pour lame conduisant ledit mécanisme de piqûre à lame situé en bordure de façon à se déplacer le long d'elle-même afin de faire en sorte que ladite lame s'étende vers l'extérieur depuis ladite ouverture de piqûre de ladite coque et de générer une coupe en forme de V ou en forme d'arc.

5. Le dispositif de piqûre selon la revendication 4, dans lequel ledit mécanisme de déclenchement et d'actionnement utilise un montant de guidage d'actionnement agencé à côté dudit bouton-poussoir de déclenchement, qui est en engagement avec ladite fente de guidage de déverrouillage de ladite coque de façon à changer lesdits états/lesdites positions dudit bouton-poussoir de déclenchement le long dudit trajet de coulissement ; et une longueur selon laquelle ledit bouton-poussoir de déclenchement est exposé à l'extérieur ou une distance entre ledit bouton-poussoir de déclenchement et ladite coque est modifiable étant donné que ladite fente de guidage de déverrouillage est configurée de telle sorte qu'une distance entre ladite fente de guidage de déverrouillage et une périphérie de ladite coque est inégale ; et ladite longueur selon laquelle ledit bouton poussoir est exposé à l'extérieur est courte et le bouton est difficile à toucher dans ledit état de verrouillage assuré(e), tandis que ladite longueur est longue dans ledit état de déclenchement.

6. Le dispositif de piqûre selon l'une quelconque des revendications 1 à 4, dans lequel ledit bouton-poussoir dudit mécanisme de déclenchement et d'actionnement emploie un bloc de poussée libérable à une extrémité distale de luimême ; lorsque ledit bouton-poussoir de déclenchement est enfoncé dans ledit état de déclenchement, ledit bloc de poussée libérable comprime et se relie audit bras d'actionnement de manière à libérer ledit trou d'arbre ou ledit crochet en forme de U vis-à-vis dudit arbre de rotation et une énergie d'actionnement est ainsi générée.

7. Le dispositif de piqûre selon l'une quelconque des revendications 1 à 6, dans lequel ledit mécanisme de piqûre à lame situé en bordure comprend une paire de protubérances de guidage de piqûre et un bloc de frappe actionné, ladite paire de protubérances de guidage de piqûre venant en engagement avec ladite paire de fentes de guidage de dispositif de piqûre à lame situé en bordure, respectivement ; l'une desdites fentes de guidage de dispositif de piqûre à lame situé en bordure est définie de façon à avoir une forme approximative d'une courbe et l'autre desdites fentes de guidage de dispositif de piqûre à lame situé en bordure est définie de façon à avoir une forme approximativement en ligne droite ; et lorsque ledit bras d'actionnement dudit mécanisme de déclenchement et d'actionnement est relâché, ladite énergie potentielle élastique fait en sorte que ledit bras d'actionnement pousse ledit bloc de frappe actionné dudit mécanisme de piqûre à lame situé en bordure de façon à se déplacer, et, ensuite, les protubérances de ladite paire de protubérances de guidage de piqûre sont entraînées de façon à s'engager et à se déplacer dans des fentes de guidage de piqûre à lame en bordure correspondantes, de sorte qu'une rotation et une translation de ladite lame sont accomplies et terminées.

8. Le dispositif de piqûre selon l'une quelconque des revendications 1 à 7, dans lequel ledit bouton-poussoir a un barbillon anti-éjection formé sur lui et ladite coque a un bossage anti-éjection formé sur elle de façon venir en engagement avec ledit barbillon anti-éjection, et se verrouiller avec lui, lorsque ladite action de déclenchement dudit bouton-poussoir de déclenchement est accomplie.

9. Le dispositif de piqûre selon l'une quelconque des revendications 1 à 8, comprenant en outre un capuchon de protection de lame, qui peut être déchiré ou cassé pourouverture, pour, de cette façon, se détacher de ladite lame.
